Europäisches Patentamt

European Patent Office

Office européen des brevets

(11) Veröffentlichungsnummer: **0 242 752**
**A2**

(12) **EUROPÄISCHE PATENTANMELDUNG**

(21) Anmeldenummer: **87105416.9**

(22) Anmeldetag: **11.04.87**

(51) Int. Cl.⁴: **A61K 37/02** , A61K 37/24

(30) Priorität: **18.04.86 DE 3613166**

(43) Veröffentlichungstag der Anmeldung:
**28.10.87 Patentblatt 87/44**

(84) Benannte Vertragsstaaten:
**AT BE CH DE ES FR GB IT LI NL SE**

(71) Anmelder: **BASF Aktiengesellschaft**
**Carl-Bosch-Strasse 38**
**D-6700 Ludwigshafen(DE)**

(72) Erfinder: **Urbaschek, Renate, Dr.**
**Trübnerstrasse 39**
**D-6900 Heidelberg(DE)**
Erfinder: **Urbaschek, Bernhard, Prof. Dr.**
**Trübnerstrasse 39**
**D-6900 Heidelberg(DE)**
Erfinder: **Maennel, Daniela, Dr.**
**Am Kirchweg 2**
**D-6901 Gaiberg(DE)**

(54) **Verwendung von TNF zur Herstellung von Arzneimitteln.**

(57) Es wird die Verwendung von TNF bei der Bekämpfung von Erkrankungen, die mit erniedrigten Leukozyten-und/oder Knochenmarkzellzahlen bzw. -funktionen einhergehen.

EP 0 242 752 A2

## Verwendung von TNF zur Herstellung von Arzneimitteln

TNF (Tumornekrosefaktor) ist eine Tumor-zerstörende Substanz, die zur Bekämpfung maligner Tumoren Verwendung finden soll. Die Struktur dieser Substanz ist in Natur 312 , 724 (1984) beschrieben worden.

Es wurde nun gefunden, daß sich TNF auch zur Behandlung von Erkrankungen eignet, die mit erniedrigten Leukozyten-und/oder Knochenmarkzellzahlen bzw. -funktionen einhergehen.

Gegenstand der Erfindung ist die Verwendung von TNF zur Herstellung von Arzneimitteln gegen Erkrankungen, die mit erniedrigten Leukozyten-und/oder Knochenmarkzellzahlen bzw. -funktionen einhergehen bzw. die Verwendung von TNF bei der Bekämpfung von Erkrankungen, die mit erniedrigten Leukozyten-und/oder Knochenmarkzellzahlen bzw. -funktionen einhergehen.

Erniedrigte Leukozyten-und/oder Knochenmarkzellzahlen bzw. -funktionen werden insbesondere gefunden nach Bestrahlung ($\alpha$-, $\beta$-, $\gamma$-oder Röntgen-Strahlen), Behandlung mit Chenotherapeutika (z.B. Alkylantien, Antimetabolite), Knochenmarkstransplantationen, bei aplastischer Anämie, bei Agranulozytosen und nach Infektionskrankheiten.

Da der TNF ein Polypeptid darstellt, welches im Magen-Darm-Trakt zerstört wird, kann er nur parenteral, vorzugsweise intavenös, verabfolgt werden. Hierzu eignen sich sterile isotonische Lösungen. Diese lassen sich beispielsweise herstellen, indem man den TNF in einer blutisotonischen wäßrigen Lösung löst, die Lösung steril filtriert und in Ampullen abfüllt. Der pH-Wert der Lösung liegt vorzugsweise zwischen 5 und 8, insbesondere bei etwa 7,5.

Die zu applizierende Dosis liegt bei 0,1 bis 5 mg, vorzugsweise 0,5 bis 3 mg TNF pro Patient und Tag. Die Behandlungsdauer beträgt in der Regel 1 bis 6 Tage.

Die therapeutische Wirksamkeit des TNF ließ sich beispielsweise wie folgt zeigen:

C3H/HEJ-und NMRI-Mäuse wurden einmal mit 0,5 $\mu$g TNF pro Tier i.v. behandelt. Zeitabhängig nach Substanzgabe kann es zu einer dosisabhängigen Erhöhung der "colony stimulating activity" (CSA), der neutrophilen Granulozyten und hämatopoetischen Stammzellen.

Herstellung einer Applikationsform

100 mg TNF werden in 300 ml 20 mM Natriumphosphatpuffer ph 7,5 gelöst. Die Lösung wird mit Kochsalz blutisotonisch eingestellt, über einen Porenfilter (Porengröße 0,1 bis 0,2 $\mu$) steril filtriert und jeweils 5 ml steril in Ampullen gefüllt.

**Ansprüche**

Verwendung von TNF zur Herstellung von Arzneimitteln zur Bekämpfung von Erkrankungen, die mit erniedrigten Leukozyten-und/oder Knochenmarkzellzahlen bzw. -funktionen einhergehen.